(19) Europäisches Patentamt
European Patent Office
Office européen des brevets

(11) EP 4 104 870 A1

(12) EUROPÄISCHE PATENTANMELDUNG

(43) Veröffentlichungstag:
**21.12.2022 Patentblatt 2022/51**

(21) Anmeldenummer: **21179463.1**

(22) Anmeldetag: **15.06.2021**

(51) Internationale Patentklassifikation (IPC):
***A61L 24/00*** *(2006.01)* ***A61L 24/04*** *(2006.01)*

(52) Gemeinsame Patentklassifikation (CPC):
(C-Sets verfügbar)
**A61L 24/001; A61L 24/046** (Forts.)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Adhesys Medical GmbH**
**52078 Aachen (DE)**

(72) Erfinder:
- **HAGEMEISTER, Kerstin**
  **52388 Nörvenich (DE)**
- **OELLERS, Nadja**
  **50933 Köln (DE)**
- **ROSENBERG, Marius**
  **40217 Düsseldorf (DE)**

(74) Vertreter: **Davepon, Björn**
**Patentanwaltskanzlei Davepon**
**Schloss Dyck**
**41363 Jüchen (DE)**

# (54) GEWEBEKLEBER IN FORM EINES ZWEIKOMPONENTEN-KLEBSTOFFS ZUR VERWENDUNG ALS BAKTERIELLE BARRIERE BEI DER WUNDBEHANDLUNG, INSBESONDERE NACH CHIRURGISCHEN EINGRIFFEN

(57) Die vorliegende Erfindung betrifft einen Gewebekleber in Form eines Zweikomponenten-Klebstoffs mit wenigstens einem aminofunktionellen Asparaginsäureester als Härter als erste Komponente und einem isocyanatfunktionellen Präpolymer als zweite Komponente zur Verwendung als bakterielle Barriere bei der Wundbehandlung, insbesondere nach chirurgischen Eingriffen. Die Erfindung betrifft zudem ein Kit aus diesem Gewebekleber und einem chirurgischen Nahtmaterial.

EP 4 104 870 A1

(52) Gemeinsame Patentklassifikation (CPC): (Forts.)

C-Sets
**A61L 24/046, C08L 75/02**

## Beschreibung

Die vorliegende Erfindung betrifft einen Gewebekleber in Form eines Zweikomponenten-Klebstoffs mit wenigstens einem aminofunktionellen Asparaginsäureester als Härter als erste Komponente und einem isocyanatfunktionellen Präpolymer als zweite Komponente zur Verwendung als bakterielle Barriere bei der Wundbehandlung, insbesondere nach chirurgischen Eingriffen. Die Erfindung betrifft zudem ein Kit aus diesem Gewebekleber und einem chirurgischen Nahtmaterial.

## Stand der Technik:

Bei der Wundversorgung werden wird in der Chirurgie oftmals ein Verschließen der Wunde über Nähen oder Klammern vorgenommen. Diese Befestigungsmethoden sind zwar im medizinischen Bereich weit verbreitet, jedoch mit verschiedenen Nachteilen behaftet. So werden sowohl durch Nähen als auch durch Klammern zusätzliche Gewebeschädigungen hervorgerufen. Zudem erhöhen die verwendeten Befestigungsmittel wie Fäden und Klammern das Fremdkörpergefühl an der operierten Stelle. Ferner muss das Nahtmaterial beziehungsweise die Klammern zu einem späteren Behandlungstermin nochmals entfernt werden, was wiederum zu kleinen Verletzungen führt. Ein weiterer Nachteil kann darin gesehen werden, dass die Fixierung des Gewebes punktuell oder allenfalls abschnittsweise erfolgt, was die mechanischen Unterstützungseigenschaften des Gewebes mindert.

Ausgehend hiervon besteht eine Weiterentwicklung dieser Behandlungsmethode darin, anstelle des Nähens oder Klammerns die Wundränder mittels eines chirurgischen Klebstoffs auf Basis von Cyanacrylaten in der Regel vollflächig zu befestigen. Ein hierfür gebräuchlicher Klebstoff ist beispielsweise Dermabond ® von der Firma Ethicon, ein Klebstoff auf Basis von 2-Octyl-Cyanoacrylat. Dieser Klebstoff bietet zudem den Vorteil, auch über einen gewissen Zeitraum als bakterielle Barriere zu fungieren, sodass eine weitere Wundabdeckung oftmals nicht erforderlich ist.

Im Rahmen von Untersuchungen, die zu der vorliegenden Erfindung geführt haben, hat sich allerdings gezeigt, dass bei der Verwendung von Cyanacrylaten als Gewebeklebstoff nicht immer eine zufriedenstellende Abheilung erfolgte. Zudem reagieren Cyanacrylate mit dem Wasser der Gewebsflüssigkeiten, was zu einem Hitzegefühl im Bereich der Wunde führen kann. Zudem wird von Patienten oftmals von einem Brennen bei der Wundbehandlung berichtet. Ein weiteres Problem der Gewebeklebstoffe auf Basis von Cyanacrylaten ist, dass diese oftmals sehr niedrigviskos sind. Dies führt bei der Applikation oftmals dazu, dass nicht nur die Wunde, sondern auch die angrenzende Haut mit dem Klebstoff überzogen wird der sich nach der schnellen Aushärtung nicht mehr ohne Weiteres entfernen lässt. Wegen der sehr kurzen Aushärtungszeit kann überschüssiger Klebstoff oftmals auch nicht mehr durch Abwischen entfernt werden.

## Aufgabe:

Die Aufgabe der vorliegenden Erfindung bestand somit darin, einen verbesserten Gewebekleber zur Verwendung als bakterielle Barriere bei der Wundbehandlung zur Verfügung zu stellen, der zum einen gute Handhabungseigenschaften aufweist und insbesondere die vorgenannten Nachteile von Gewebeklebstoffen auf Basis von Cyanacrylaten nicht aufweist und zugleich gute bakterielle Barriere bildet, insbesondere innerhalb der ersten 48 Stunden nach der Applikation, bevorzugt innerhalb der ersten 72 Stunden nach Applikation.

## Lösung:

Die Aufgabe wird gelöst durch einen Gewebekleber in Form eines Zweikomponenten-Klebstoffs mit wenigstens einem aminofunktionellen Asparaginsäureester als Härter als erste Komponente und einem isocyanatfunktionellen Präpolymer als zweite Komponente zur Verwendung als bakterielle Barriere bei der Wundbehandlung, insbesondere nach chirurgischen Eingriffen.

Der Erfindung liegt die Erkenntnis zugrunde, dass bei Einsatz des erfindungsgemäßen Gewebeklebers in Form eines Zweikomponenten-Klebstoffs ("Klebstoff-System") bei der Wundbehandlung einerseits ein sehr schneller Verschluss der Operationsöffnung erreicht werden kann und beispielsweise innerhalb von 2 bis 3 Minuten die operierte Stelle verschlossen ist. Laborversuche haben zudem gezeigt, dass der erfindungsgemäße Zweikomponenten-Klebstoff innerhalb der ersten 48 Stunden nach der Applikation gute bakterielle Barriere-Eigenschaften aufweist, bevorzugt innerhalb der ersten 72 Stunden nach Applikation. Nach dieser Zeitspanne ist die Wunde in der Regel schon so weit verheilt, dass das Infektionsrisiko stark verringert ist. Dementsprechend verheilen die mit dem erfindungsgemäßen Klebstoff versorgten Wunden ausreichend schnell, ohne dass Entzündungen auftreten, die zu einer schlechteren Wundheilung führen und gegebenenfalls eine separate Nachbehandlung erforderlich machen.

Ein weiterer Vorteil der erfindungsgemäßen Lösung besteht darin, dass der erfindungsgemäße Zweikomponenten-Klebstoff auch mit chirurgischem Nahtmaterial kompatibel ist, der erfindungsgemäße Gewebekleber also zusammen mit herkömmlichen chirurgischen Methoden zur Anwendung kommen kann.

Zudem zeichnen sich die erfindungsgemäßen Zweikomponenten-Klebstoffe durch eine gute biologische Abbaubarkeit aus, die je nach Menge der Lactidgruppen beziehungsweise des Co-Monomergehalts entsprechend der EP 2 794 710 B1 beispielsweise bei 4 Wochen bis 12 Monaten liegen kann.

Ein weiterer Vorteil des erfindungsgemäßen Zweikomponenten-Klebstoffs liegt darin, dass der ausgehärtete Gewebekleber gemäß ISO 10993-5:2009 keine Zytotoxizität aufweist.

Erfindungsgemäß bevorzugte Klebstoffe sind beispielsweise solche, wie sie in EP 2 307 063 B1, EP 2 145 634 B1 und EP 2 794 710 B1 offenbart sind. Besonders bevorzugt solche der Beispiele 1 und 2 der EP 2 145 634 B1 sowie des Beispiels Polyol 1 aus und Präpolymer 2 der EP 2 794 710 B1 und einem Härter aus Aspartat A/Aspartat B/PEG 200 im Verhältnis von 0,369/0,098/0,53. Ebenfalls bevorzugt sind Klebstoffe gemäß Beispiel 3 der EP 2 307 063 B1. Die Offenbarung der EP 2 145 634 B1, der EP 2 307 063 B1 und EP 2 794 710 B1 wird hiermit durch Bezugnahme in die vorliegende Offenbarung einbezogen.

Nach einer bevorzugten Ausführungsform des erfindungsgemäßen Gewebeklebers umfasst oder besteht der Gewebekleber aus den folgenden Komponenten:

ein isocyanatfunktionelles Präpolymer als Komponente A), erhältlich durch

a) Umsetzung einer wenigstens ein Zerewitinoff-aktives H-Atom aufweisenden H-funktionellen Starterverbindung mit einer Alkylenoxidverbindung und einem Co-Monomer zu einer Hydroxylgruppen tragenden Vorstufe, wobei das Co-Monomer ausgewählt ist aus der Gruppe umfassend Lactide, Glycolide, sowie Kombinationen hiervon und Kombinationen von Lactiden und / oder Glycoliden mit cyclischen Dicarbonsäureanhydriden und wobei das Co-Monomer durch eine statistische Copolymerisation in die Polymerkette(n) der Hydroxylgruppen tragenden Vorstufe eingebaut ist, sowie

b) Umsetzung der Hydroxylgruppen tragenden Vorstufe aus Schritt a) mit einem polyfunktionellen Isocyanat zu dem isocyanatfunktionellen Präpolymer,

ein aminofunktioneller Asparaginsäureester als Komponente B) der allgemeinen Formel (I)

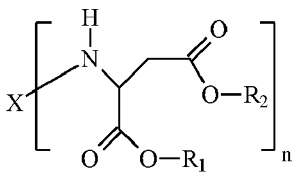

(I) ist,

wobei

X ein n-wertiger organischer Rest ist,

R1, R2 gleiche oder verschiedene organische Reste sind, die keine Zerewitinoff-aktiven H-Atome aufweisen,

n eine ganze Zahl ≥ 2 ist,

sowie optional ein Umsetzungsprodukt des isocyanatfunktionellen Präpolymers A) mit dem aminofunktionellen Asparaginsäureester B) als Komponente C).

Mit anderen Worten enthält das erfindungsgemäße isocyanatfunktionelle Präpolymer in den Polymerketten Estergruppen, die durch statistische Copolymerisation von Alkylenoxidverbindungen und Lactiden, Glycoliden und / oder cyclischen Dicarbonsäureanhydriden auf Zerewitinoff-aktive H-Atome enthaltende Starterverbindungen erzeugt werden. Insbesondere sind die Estergruppen nicht blockweise eingebaut. Dabei ist unter dem unbestimmten Artikel "ein", "einer" usw. zu verstehen, dass wahlweise jeweils auch mehrere dieser Komponenten miteinander umgesetzt werden können. Die genannten Komponenten, insbesondere das Co-Monomer, kann auch dimer, trimer usw. eingesetzt werden, wie beispielsweise als Dilactid.

Solche isocyanatfunktionellen Präpolymere sind im Körper eines Patienten biologisch abbaubar. Die Abbauzeit liegt dabei oberhalb der Heilungsdauer für die zu verschließende Wunde, beispielsweise bei 4 Wochen. Hierbei scheint sich insbesondere die statistische Verteilung der Co-Monomereinheiten in der (den) Polymerkette(n) vorteilhaft auf die Abbaugeschwindigkeit auszuwirken, da diese als "Sollbruchstellen" in dem ausgehärteten Klebstoff fungieren. Wenn die Co-Monomerbausteine beim biologischen Abbau angegriffen und aufgebrochen werden, verkürzt sich dadurch die

Polymerkettenlänge besonders schnell.

Gleichzeitig zeichnen sich die erfindungsgemäßen isocyanatfunktionellen Präpolymere durch eine hohe Adhäsion insbesondere auf menschlichem oder tierischem Gewebe sowie eine hohe Aushärtungsgeschwindigkeit aus. Zudem erfüllen Gewebekleber mit einem erfindungsgemäßen isocyanatfunktionellen Präpolymer die Anforderungen hinsichtlich der Histotoxizität und Thrombogenität.

Im Rahmen der vorliegenden Erfindung ist vorgesehen, dass die H-funktionelle Starterverbindung wenigstens ein Zerewitinoff-aktives H-Atom trägt. Unter einem Zerewitinoff-aktiven H-Atom wird im Rahmen der vorliegenden Erfindung ein azides H-Atom oder "aktives" H-Atom verstanden. Ein solches kann in an sich bekannter weise durch eine Reaktion mit einem entsprechenden Grignard-Reagenz ermittelt werden. Die Menge an Zerewitinoff-aktiven H-Atomen wird typischerweise über die Methanfreisetzung gemessen, die bei einer Reaktion der zu überprüfenden Substanz mit Methylmagnesiumbromid (CH3-MgBr) gemäß der folgenden Reaktionsgleichung frei wird:

$$CH_3\text{-}MgBr + ROH \rightarrow CH_4 + Mg\,(OR)Br$$

Zerewitinoff-aktive H-Atome stammen typischerweise von C-H aziden organischen Gruppen, -OH, -SH, -NH2 oder -NHR mit R als organischem Rest sowie -COOH.

Besonders geeignete H-funktionelle Starterverbindungen besitzen eine H-Funktionalität von 1 bis 35, insbesondere 1 bis 16, bevorzugt 1 bis 8, wobei sich die H-Funktionalität auf die vorgenannten Zerewitinoff-aktiven H-Atome bezieht.

Als H-funktionelle Starterverbindungen sind insbesondere polyhydroxyfunktionelle Polymere geeignet, welche insbesondere ausgewählt sind aus geradkettigen und/oder verzweigten Polyethern, Polyestern, Polyetherpolyestern, Polycarbonaten, Polyetherpolycarbonaten, sowie Kombinationen hiervon.

Sofern das als H-funktionelle Starterverbindung eingesetzte Hydroxylgruppen tragende Polymer ein Polyether ist oder Polyethergruppen aufweist, enthalten diese weiter bevorzugt Ethylenoxideinheiten, wobei der Gewichtsanteil der Ethylenoxideinheiten in einem solchen vorgefertigten Alkylenoxidadditionsprodukt insbesondere wenigstens 40 Gew.% beträgt, bevorzugt wenigstens 50 Gew.%. Beispielsweise beträgt der Gewichtsanteil an Ethylenoxideinheiten 40 bis 90 Gew.-%, bevorzugt 50 bis 80 Gew.-%, jeweils bezogen auf die Masse des Hydroxylgruppen tragenden Polymers. Der Rest des Polyethergerüsts bzw. der Polyetherbausteine kann jeweils durch andere Alkylenoxideinheiten wie insbesondere (Poly)propylenoxid, (Poly)butylenoxid oder andere (Poly)Alkylenoxidgruppen und Mischungen hiervon aufgebaut sein. Die Molekulargewichte der H-funktionellen Starterverbindung können über weite Bereiche variieren. So kann das mittlere Molgewicht beispielsweise 17 bis 10000 g/mol betragen, insbesondere von mehr als 200 bis 9000 g/mol. Das mittlere Molgewicht bezeichnet bei polymeren Verbindungen deren Zahlenmittel, welches über an sich bekannte Methoden, beispielsweise über Gelpermeationschromatographie oder die Ermittlung der OH-Zahl bestimmbar ist. Mit anderen Worten kann als H-funktionelle Starterverbindung für das erfindungsgemäße Präpolymer eine monomere Starterverbindung gewählt werden, wie beispielsweise Ammoniak oder Ethylenglykol. Auch oligomere Starterverbindungen sind umfasst, beispielsweise Polyether mit einem mittleren Molgewicht von 200 bis 600 g/mol sowie polymere Starterverbindungen mit höheren Molekulargewichten, beispielsweise von mehr als 600 bis 10000 g/mol oder 800 bis 9000 g/mol.

Neben den bevorzugt zu verwendenden hydroxyfunktionellen Startern können auch aminofunktionelle Starter eingesetzt werden. Beispiele für hydroxyfunktionelle Starterverbindungen sind Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, sowie methylolgruppenhaltige Kondensate aus Formaldehyd und Phenol oder Harnstoff. Es können auch hochfunktionelle Starterverbindungen auf Basis von hydrierten Stärkehydrolyseprodukten eingesetzt werden. Solche sind beispielsweise in EP 1525244 A1 beschrieben.

Beispiele für aminogruppenhaltige H-funktionelle Starterverbindungen sind Ammoniak, Ethanolamin, Diethanolamin, Triethanolamin, Isopropanolamin, Diisopropanolamin, Ethylendiamin, Hexamethylendiamin, Anilin, die Isomere des Toluidins, die Isomere des Diaminotoluols, die Isomere des Diaminodiphenylmethans sowie bei der Kondensation von Anilin mit Formaldehyd zu Diaminodiphenylmethan anfallende höherkernige Produkte, ferner methylolgruppenhaltige Kondensate aus Formaldehyd und Melamin sowie Mannichbasen. Außerdem können als Starterverbindungen auch Ringöffnungsprodukte aus cyclischen Carbonsäureanhydriden und Polyolen eingesetzt werden. Beispiele sind Ringöffnungsprodukte aus Phthalsäureanhydrid oder Bernsteinsäureanhydrid einerseits und Ethylenglykol, Diethylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit oder Sorbit andererseits. Daneben ist es auch möglich, ein- oder mehrfunktionelle Carbonsäuren direkt als Starterverbindungen einzusetzen.

Ferner können in dem Prozess auch vorgefertigte Alkylenoxidadditionsprodukte der erwähnten Starterverbindungen, also Polyetherpolyole vorzugsweise mit OH-Zahlen von 5 bis 1000 mg KOH/g, bevorzugt 10 bis 1000 mg

KOH/g, als Starterverbindungen eingesetzt, bzw. dem Reaktionsgemisch zugesetzt werden. Auch ist es möglich, im erfindungsgemäßen Prozess Polyesterpolyole vorzugsweise mit OH-Zahlen im Bereich von 6 bis 800 mg KOH/g als Starter oder Co-Starter einzusetzen. Hierfür geeignete Polyesterpolyole können beispielsweise aus organischen Dicarbonsäuren mit 2 bis 12 Kohlenstoffatomen und mehrwertigen Alkoholen, vorzugsweise Diolen, mit 2 bis 12 Kohlenstoffatomen, vorzugsweise 2 bis 6 Kohlenstoffatomen nach bekannten Verfahren hergestellt werden.

Des Weiteren können als H-funktionelle Startersubstanzen Polycarbonatpolyole, Polyestercarbonatpolyole oder Polyethercarbonatpolyole, bevorzugt Polycarbonatdiole, Polyestercarbonatdiole oder Polyethercarbonatdiole vorzugsweise jeweils mit OH-Zahlen im Bereich von 6 bis 800 mg KOH/g, als Starter oder Co-Starter verwendet werden. Diese werden beispielsweise durch Umsetzung von Phosgen, Dimethylcarbonat, Diethylcarbonat oder Diphenylcarbonat mit di- oder höherfunktionellen Alkoholen oder Polyesterpolyolen oder Polyetherpolyolen hergestellt.

Eingesetzt werden können auch Polyethercarbonatpolyole, wie sie beispielsweise durch katalytische Umsetzung von Alkylenoxiden (Epoxiden) und Kohlendioxid in Anwesenheit von H-funktionellen Startersubstanzen erhältlich sind (siehe z.B. EP-A 2046861). Diese Polyethercarbonatpolyole haben bevorzugt eine OH-Zahl von $\geq$ 5 mg KOH / g bis $\leq$ 240 mg KOH/g, besonders bevorzugt von $\geq$ 9 bis $\leq$ 200 mg KOH/g.

In Schritt a) der Herstellung des erfindungsgemäßen Präpolymers dienen bevorzugt aminogruppenfreie H-funktionelle Starterverbindungen mit Hydroxylgruppen als Träger der aktiven Wasserstoffe wie beispielsweise Methanol, Ethanol, 1-Propanol, 2-Propanol und höhere aliphatische Monole, insbesondere Fettalkohole, Phenol, alkylsubstituierte Phenole, Propylenglykol, Ethylenglykol, Diethylenglykol, Dipropylenglykol, 1,2-Butandiol, 1,3-Butandiol, 1,4-Butandiol, Hexandiol, Pentandiol, 3-Methyl-1,5-pentandiol, 1,12-Dodecandiol, Glycerin, Trimethylolpropan, Pentaerythrit, Sorbit, Saccharose, Hydrochinon, Brenzcatechin, Resorcin, Bisphenol F, Bisphenol A, 1,3,5-Trihydroxybenzol, methylolgruppenhaltige Kondensate aus Formaldehyd und Phenol und hydrierte Stärkehydrolyseprodukte. Es können auch Gemische verschiedener H-funktioneller Starterverbindungen eingesetzt werden.

Als erfindungsgemäß verwendbare Alkylenoxidverbindungen können solche Vertreter ausgewählt sein, die 2 bis 24 Kohlenstoffatome aufweisen, insbesondere 2 bis 12 Kohlenstoffatome, weiter bevorzugt 2 bis 6 Kohlenstoffatome, sowie die Kombination unterschiedlicher Alkylenoxidverbindungen der vorgenannten Art. Bei den Epoxiden mit 2 bis 24 Kohlenstoffatomen handelt es sich beispielsweise um eine oder mehrere Verbindungen ausgewählt aus der Gruppe bestehend aus Ethylenoxid, Propylenoxid, 1-Butenoxid, 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 1-Hexenoxid, 2,3-Hexenoxid, 3,4-Hexenoxid, 2-Methyl-1,2-pentenoxid, 4-Methyl-1,2-pentenoxid, 2-Ethyl-1,2-butenoxid, 1-Heptenoxid, 1-Octenoxid, 1-Nonenoxid, 1-Decenoxid, 1-Undecenoxid, 1-Dodecenoxid, 4-Methyl-1,2-pentenoxid, Butadienmonoxid, Isoprenmonoxid, Cyclopentenoxid, Cyclohexenoxid, Cycloheptenoxid, Cyclooctenoxid, Styroloxid, Methylstyroloxid, Pinenoxid, ein- oder mehrfach epoxidierte Fette als Mono-, Di- und Triglyceride, epoxidierte Fettsäuren, C1-C24-Ester von epoxidierten Fettsäuren, Epichlorhydrin, Glycidol, und Derivate des Glycidols wie beispielsweise Methylglycidylether, Ethylglycidylether, 2-Ethylhexylglycidylether, Allylglycidylether, Glycidylmethacrylat sowie epoxidfunktionelle Alkyloxysilane wie beispielsweise 3-Glycidyloxypropyltrimethoxysilan, 3-Glycidyloxypropyltriethoxysilan, 3-Glycidyloxypropyltripropoxysilan, 3-Glycidyloxypropyl-methyl-dimethoxysilan, 3-Glycidyloxypropylethyldiethoxysilan und 3-Glycidyloxypropyltriisopropoxysilan. Vorzugsweise werden Ethylenoxid und/oder Propylenoxid verwendet. Insbesondere kann der Gewichtsanteil an Ethylenoxid, bezogen auf die Gesamtmasse der dosierten Alkylenoxidverbindungen, wenigstens 40 Gew.-% betragen, bevorzugt wenigstens 50 Gew.-%. Beispielsweise beträgt der Gewichtsanteil an Ethylenoxid 40 - 90 %, bevorzugt 50 - 80 %, jeweils bezogen auf die Gesamtmasse der dosierten Alkylenoxidverbindungen.

Es ist erfindungsgemäß vorgesehen, dass das isocyanatfunktionelle Präpolymer Bausteine herrührend von Lactiden, Glycoliden und / oder cyclischen Dicarbonsäureanhydriden enthält, die durch eine statistische Copolymerisation in die Polymerkette der Hydroxylgruppen tragenden Vorstufe eingebaut sind. In bevorzugter Weise beträgt das Stoffmengenverhältnis der Alkylenoxidverbindung zu diesem Co-Monomer in der Hydroxylgruppen tragenden Vorstufe 200 : 1 bis 1 : 1 beträgt, insbesondere 10 : 1 bis 5 : 1. Diese Stoffmengenverhältnisse sind besonders bevorzugt, weil ein Gewebekleber enthaltend eine solche Präpolymervorstufe ein gutes Adhäsionsvermögen bei geringer Aushärtezeit besitzt und sich zudem unter physiologischen Bedingungen schnell abbaut.

Grundsätzlich ist es auch möglich, weitere Co-Monomere, wie z.B. cylische Anhydride oder Kohlendioxid durch statistische Copolymerisation in die Polymerkette des Hydroxylgruppen tragenden Präpolymers mit einzubauen.

Die Herstellung der Komponente A) kann unkatalysiert durchgeführt werden, wobei der Einsatz eines Katalysators jedoch bevorzugt ist. Hierbei kann insbesondere der Schritt a) über einen Doppelmetallcyanid-Katalysator (DMC-Katalysator) katalysiert werden, der insbesondere Zinkhexacyanocobaltat (III), Zinkhexacyanoiridat (III), Zinkhexacyanoferrat (III) oder Cobalt(II) hexacyanocobaltat (III) enthält. Ein besonderer Vorteil dieser Ausgestaltung liegt darin, dass die als Zwischenprodukt des Schrittes a) erhaltene Hydroxylgruppen tragende Vorstufe eine vergleichbar enge Molekülkettenlängen-Verteilung aufweist. Einer der Gründe kann in dem Einsatz der DMC-Katalyse gesehen werden, denn solche Katalysatoren zeigen eine sogenannte "catch-up"-Kinetik. Das heißt, dass mit zunehmender Kettenlänge die katalytische Aktivität für die Anbindung des nächsten Monomerbausteins sukzessive abnimmt und damit auch die Reaktionsgeschwindigkeit.

Wie vorstehend erläutert wird als bevorzugter aminofunktionelle Asparaginsäureester ein solcher der allgemeinen Formel (I) eingesetzt. Weiter bevorzugt ist ein aminofunktioneller Asparaginsäureester der allgemeinen Formel (II)

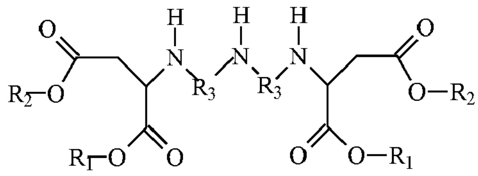

(II),

wobei R1, R2, R3 gleiche oder verschiedene organische Reste sind, die keine Zerewitinoff-aktiven H-Atome aufweisen.

Nach einer besonders bevorzugten Ausführungsform des erfindungsgemäßen Gewebeklebers ist das zur Herstellung der Hydroxylgruppen tragenden Vorstufe in Schritt a) eingesetzte Co-Monomer ein Laktid, bevorzugt Dilaktid, und der Gehalt an Laktidgruppen in der Hydroxylgruppen tragenden Vorstufe beträgt 2 bis 22 Gew.-%, vorzugsweise 3 bis 15 Gew.-%, weiter bevorzugt 4 bis 10 Gew.-%, besonders bevorzugt 6 bis 9 Gew.-%, wobei der Gehalt an Laktidgruppen in der Hydroxylgruppen tragenden Vorstufe folgendermaßen definiert ist:

Gehalt Laktid in der Hydroxylgruppen tragenden Vorstufe (in Gew.-%):

$$m(Laktid)*100/[m(Laktid)+m(Kat)+m(EO)+m(PO)+m(Starter)]$$

Wird Dilaktid eingesetzt, lautet die Formel folglich

$$m(Dilaktid)*100/[m(Dilaktid)+m(Kat)+m(EO)+m(PO)+m(Starter)]$$

Bei Gewebeklebern aus den Komponenten A), B) sowie optional Umsetzungsprodukten des isocyanatfunktionellen Präpolymers A) mit dem aminofunktionellen Asparaginsäureester B) als Komponente C) sind die vorgenannten Gehalte an Laktid besonders vorteilhaft, weil sich diese überraschenderweise positiv auf die bakterielle Barrierefunktion auswirken.

Zur Verbesserung der biologischen Kompatibilität ist das polyfunktionelle Isocyanat vorzugsweise ausgewählt aus aliphatischen Isocyanaten, insbesondere aus Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat, Diisocyanatododecan oder Kombinationen hiervon. Hierbei sind Hexamethylendiisocyanat (HDI), Isophorondiisocyanat (IPDI), Butylendiisocyanat (BDI) und Bis(isocyanatocyclohexyl)methan (HMDI) bevorzugt. Besonders bevorzugt sind Hexamethylendiisocyanat, Isophorondiisocyanat, Butylendiisocyanat, ganz besonders bevorzugt Hexamethylendiisocyanat und Isophorondiisocyanat.

Grundsätzlich ist die Erfindung jedoch nicht auf den Einsatz aliphatischer Isocyanate beschränkt, es können also auch übliche aromatische Isocyanate verwendet werden, wie beispielsweise Toluol-diisocyanat (TDI) oder Diphenylmethandiisocyanat (MDI).

Die Umsetzung des nach Schritt a) erhaltenen Hydroxylgruppen tragenden Präpolymers mit dem polyfunktionellen Isocyanat in Schritt b) kann bei einem NCO/OH-Verhältnis von 4:1 bis 12:1 erfolgen, bevorzugt 8:1, und anschließend kann der Anteil an nicht umgesetztem Isocyanat mittels geeigneter Methoden abgetrennt werden. Üblicherweise wird hierfür die Dünnschichtdestillation verwendet, wobei ein Präpolymer mit einem Restmonomergehalt von weniger als 1 Gew.-%, bevorzugt weniger als 0,1 Gew.-%, ganz besonders bevorzugt weniger als 0,03 Gew.-% erhalten wird.

Gegebenenfalls können während der Herstellung des isocyanatfunktionellen Präpolymers Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Umsetzung im Schritt b) beträgt bevorzugt 20 bis 120 °C und weiter bevorzugt 60 bis 100 °C.

Das isocyanatfunktionelle Präpolymer hat bevorzugt einen nach DIN EN ISO 11909 gemessenen mittleren NCO-Gehalt von 2 bis 10 Gew.-%, bevorzugt von 2,5 bis 8 Gew.-%.

Die mittlere NCO-Funktionalität des isocyanatfunktionellen Präpolymers beträgt bevorzugt 1,5 bis 6, weiter bevorzugt 1,6 bis 5, noch weiter bevorzugt 1,7 bis 4, ganz besonders bevorzugt 1,8 bis 3,5 und insbesondere 3.

Erfindungsgemäß zeichnet sich der eingesetzte Klebstoff durch einen Aspartathärter aus. Der Gewebekleber

kann jedoch bevorzugt wenigstens einen weiteren Härter umfassen. So umfasst der erfindungsgemäße Gewebekleber-nach einer besonders bevorzugten Ausführungsform ferner einen weiteren Härter, der insbesondere aus Polyolen mit einer zahlenmittleren Molmasse von 1000 Da oder weniger ausgewählt ist, insbesondere von 600 Da oder weniger, weiter bevorzugt 400 Da oder weniger oder gar 300 Da oder weniger. Als Polyole eignen sich beispielsweise PEG oder PPG. Durch den Zusatz dieser zusätzlichen Härter kann die Aushärtungsgeschwindigkeit des erfindungsgemäßen Gewebeklebers beeinflusst, das heißt in der Regel verkürzt werden, so dass eine bedarfsgerechte Konfektionierung des Gewebeklebers erfolgen kann. Bevorzugt wird beispielweise ein Polyethylenglycol 200 verwendet.

In einer alternativen Ausführungsform des erfindungsgemäßen Gewebeklebers umfasst der Gewebekleber die folgende Formulierung aus Komponenten D) sowie E) und/ oder F) oder besteht daraus:

D) isocyanatfunktionelle Prepolymere erhältlich aus

D1) aliphatischen Isocyanaten und

D2) Polyolen mit zahlenmittleren Molekulargewichten von ≥ 400 g/mol und mittleren OH-Funktionalitäten von 2 bis 6,

E) eine Härterkomponente umfassend

E1) aminofunktionelle Asparaginsäureester der allgemeinen Formel (III)

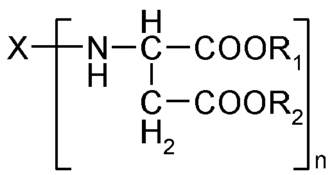

(III)

wobei

X ein n-wertiger organischer Rest ist, der durch Entfernung der primären Aminogruppen eines n-wertigen Amins erhalten wird,

$R_1$, $R_2$ gleiche oder verschiedene organische Reste sind, die keinen Zerewitinoff-aktiven Wasserstoff aufweisen und

n eine ganze Zahl von mindestens 2 ist

und gegebenenfalls

E2) organische Füllstoffe, die eine nach DIN 53019 gemessenen Viskosität bei 23°C im Bereich von 10 bis 6000 mPas aufweisen,

F) Umsetzungsprodukte von isocyanatfunktionellen Prepolymeren gemäß der Definition der Komponente D) mit Asparaginsäureestern gemäß Komponente E1) und/oder organischen Füllstoffen gemäß Komponente E2).

Die in D) eingesetzten isocyanatfunktionellen Prepolymere sind durch Umsetzung von Isocyanaten mit hydroxyfunktionellen Polyolen gegebenenfalls unter Zusatz von Katalysatoren sowie Hilfs- und Zusatzstoffen erhältlich.

In D1) können als Isocyanate beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoate (Lysindiisocyanat) mit C1-C8-Alkylgruppen eingesetzt werden.

Neben den vorstehend genannten monomeren Isocyanaten können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden in D1) Isocyanate der vorstehend genannten Art mit ausschließlich aliphatisch oder cycloaliphatisch gebundenen Isocyanatgruppen oder deren Mischungen eingesetzt.

Die in D1) eingesetzten Isocyanate oder Isocyanatmischungen haben bevorzugt eine mittlere NCO-Funktionalität von 2 bis 4, besonders bevorzugt 2 bis 2,6 und ganz besonders bevorzugt 2 bis 2,4.

In einer besonders bevorzugten Ausführungsform wird in D1) Hexamethylendiisocyanat eingesetzt.

Zum Prepolymeraufbau können in D2) grundsätzliche alle dem Fachmann an sich bekannten Polyhydroxyverbindungen mit 2 oder mehr OH-Funktionen pro Molekül eingesetzt werden. Dies können beispielsweise Polyesterpolyole, Polyacrylatpolyole, Polyurethanpolyole, Polycarbonatpolyole, Polyetherpolyole, Polyesterpolacrylatpolyole, Polyurethanpolyacrylatpolyole, Polyurethanpolyesterpolyole, Polyurethanpolyetherpolyole, Polyurethanpolycarbonat-polyole, Polyesterpolycarbonatpolyole oder deren beliebige Mischungen untereinander sein.

Die in D2) eingesetzten Polyole haben bevorzugt eine mittlere OH-Funktionalität von 3 bis 4.

Die in D2) eingesetzten Polyole haben ferner bevorzugt ein zahlenmittleres Molekulargewicht von 400 bis 20000 g/mol, besonders bevorzugt 2000 bis 10000 g/mol und ganz besonders bevorzugt 4000 bis 8500.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Bevorzugte Polyalkylenoxid-Polyether entsprechen denen der vorstehend genannten Art und weisen Gehalt an Ethylenoxid-basierten Einheiten von 50 bis 100 %, bevorzugt 60 bis 90 % bezogen auf die insgesamt enthaltene Mengen an Alkylenoxideinheiten auf.

Bevorzugte Polyesterpolyole sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können hydroxylgruppenaufweisende Polycarbonate, bevorzugt Polycarbonatdiole, mit zahlenmittleren Molekulargewichten $M_n$ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylen-glykol, Polybutylenglykole, Bisphenol A und lactonmodifizierte Diole der vorstehend genannten Art in Frage.

Bevorzugt werden zum Prepolymeraufbau Polyetherpolyole der vorstehend genannten Art eingesetzt.

Zur Herstellung des Prepolymers werden die Verbindungen der Komponente D1) mit denen der Komponente D2) bei einem NCO/OH-Verhältnis von bevorzugt 4:1 bis 20:1, besonders bevorzugt 8:1 umgesetzt und anschließend der Anteil an nicht umgesetzten Verbindungen der Komponente D1) mittels geeigneter Methoden abgetrennt. Üblicherweise wird hierfür die Dünnschichtdestillation verwendet, wobei restmonomerenarme Produkte mit Restmonomergehalten von weniger als 1 Gew.-%, bevorzugt weniger als 0,5 Gew.-%, ganz besonders bevorzugt weniger als 0,1 Gew.-% erhalten werden.

Gegebenenfalls können während oder nach der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur beträgt dabei 20 bis 120°C, bevorzugt 60 bis 100°C.

Bevorzugt sind in Formel (III):

$R_1$, $R_2$ gleiche oder verschiedene gegebenenfalls verzweigte oder cyclische organische Reste mit 1 bis 20, bevorzugt 1 bis 10 Kohlenstoffatomen, die keinen Zerewitinoff-aktiven Wasserstoff aufweisen,

n eine ganze Zahl von 2 bis 4 und

X ein n-wertiger organischer gegebenenfalls verzweigter oder cyclischer organischer Rest mit 2 bis 20, bevorzugt 5 bis 10 Kohlenstoffatomen, der durch Entfernung der primären Aminogruppen eines n-wertigen primären Amins erhalten wird.

Die Herstellung der aminofunktionellen Polyasparaginsäureester E1) erfolgt in bekannter Weise durch Umsetzung der entsprechenden primären mindestens difunktionellen Amine $X(NH_2)_n$ mit Malein- oder Fumarsäureestern der allgemeinen Formel

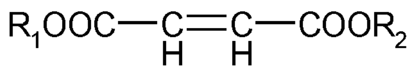

Bevorzugte Malein- oder Fumarsäureester sind Maleinsäuredimethylester, Maleinsäurediethylester, Maleinsäuredibutylester und die entsprechenden Fumarsäureester.

Bevorzugte primäre mindestens difunktionelle Amine $X(NH_2)_n$ sind Ethylendiamin, 1,2-Diaminopropan, 1,4-Diaminobutan, 1,3-Diaminopentan, 1,5-Diaminopentan, 2-Methyl-1,5-Diaminopentan, 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'und/oder 4,4'-Diamino-dicyclohexylmethan, 3,3'-Dimethyl-4,4'-diamino-dicyclohexylmethan, 2,4,4'-Triamino-5-methyl-dicyclohexylmethan und Polyetheramine mit aliphatisch gebundenen primären Aminogruppen mit einem zahlenmittleren Molekulargewicht $M_n$ von 148 bis 6000 g/mol.

Besonders bevorzugte primäre mindestens difunktionelle Amine sind 1,3-Diaminopentan, 1,5-Diaminopentan, 2-Methyl-1,5-diaminopentan, 1,6-Diaminohexan, 1,13-Diamino-4,7,10-trioxatridecan. Ganz besonders bevorzugt ist 2-Methyl-1,5-diaminopentan.

Bevorzugt sind $R_1$ und $R_2$ unabhängig voneinander $C_1$ bis $C_{10}$-Alkylreste, besonders bevorzugt Methyl oder Ethylreste.

In einer bevorzugten Ausführungsform der Erfindung sind $R_1 = R_2$ = Ethyl, wobei X auf 2-Methyl-1,5-Diaminopentan, als n-wertigem Amin basiert.

Bevorzugt ist n in Formel (III) für die Beschreibung der Wertigkeit des n-wertigen Amins eine ganze Zahl von 2 bis 6, besonders bevorzugt 2 bis 4.

Die Herstellung der aminofunktionellen Asparaginsäureester E1) aus den genannten Ausgangsmaterialien erfolgt nach DE-A 69 311 633 bevorzugt innerhalb des Temperaturbereichs von 0 bis 100 °C, wobei die Ausgangsmaterialien in solchen Mengenverhältnissen eingesetzt werden, dass auf jede primäre Aminogruppe mindestens eine, vorzugsweise genau eine olefinische Doppelbindung entfällt, wobei im Anschluss an die Umsetzung gegebenenfalls im Überschuss eingesetzte Ausgangsmaterialien destillativ abgetrennt werden können. Die Umsetzung kann in Substanz oder in Gegenwart geeigneter Lösungsmittel wie Methanol, Ethanol, Propanol oder Dioxan oder Gemischen derartiger Lösungsmittel erfolgen.

Die in E2) eingesetzten organischen flüssigen Füllstoffe sind bevorzugt gemäß Zytotoxizitätsmessung nach ISO 10993 nicht zytotoxisch.

Beispielsweise können als organische Füllstoffe flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Bevorzugt handelt es sich bei den organischen Füllstoffen der Komponente E2) um hydroxy- oder aminofunktionelle, bevorzugt rein hydroxyfunktionelle Verbindungen. Bevorzugte rein hydroxyfunktionelle Verbindungen sind Polyether- und/oder Polyesterpolyole, besonders bevorzugt Polyetherpolyole.

Die bevorzugten organischen Füllstoffe der Komponente E2) besitzen bevorzugt mittlere OH-Funktionalitäten von 1,5 bis 3, besonders bevorzugt 1,8 bis 2,2, ganz besonders bevorzugt 2,0.

Die bevorzugten organischen Füllstoffe der Komponente E2) besitzen bevorzugt sich wiederholende von Ethylenoxid abgeleitete Einheiten.

Die Viskosität der organischen Füllstoffe der Komponente E2) beträgt bevorzugt 50 bis 4000 mPas bei 23°C gemessen nach DIN 53019.

In einer bevorzugten Ausführungsform der Erfindung werden als organische Füllstoffe der Komponente E2) Polyethylenglykole eingesetzt. Diese haben bevorzugt ein zahlenmittleres Molekulargewicht von 100 bis 1000 g/mol, besonders bevorzugt 200 bis 400 g/mol.

Das Gewichtsverhältnis von E1) zu E2) beträgt 1:0 bis 1:20 , bevorzugt 1:0 bis 1:12.

Das Gewichtsverhältnis der Komponente B2 bezogen auf die Gesamtmenge der Mischung aus B1, B2 und A liegt im Bereich von 0 bis 100 %, bevorzugt 0 bis 60 %.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich zu den in E1) und E2) eingesetzten Verbindungen auch die amino- oder hydroxyfunktionellen Umsetzungsprodukte isocyanatfunktioneller Prepolymere mit Asparaginsäureestern und/oder organischen Füllstoffen E2), sofern diese amino- oder hydroxyfunktionell sind, in einer separaten Vorreaktion herzustellen und dann als höhermolekulare Härterkomponente F) einzusetzen.

Bevorzugt werden bei der Vorverlängerung Verhältnisse von isocyanatreaktiven Gruppen zu Isocyanatgruppen von 50 zu 1 bis 1,5 zu 1 eingesetzt, besonders bevorzugt 15 zu 1 bis 4 zu 1.

Das dafür einzusetzende isocyanatfunktionelle Prepolymer kann dabei demjenigen der Komponente D) entsprechen oder aber auch abweichend aus den Komponenten, wie sie als mögliche Bestandteile der isocyanatfunktionellen Prepolymere im Rahmen dieser Anmeldung gelistet sind, aufgebaut sein.

Vorteil dieses Modifizierung durch Vorverlängerung ist, dass das Equivalentgewicht und Equivalentvolumen der Härterkomponente in deutlichen Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden.

Bei Bedarf kann eine der beiden Komponenten angefärbt werden.

Die erfindungswesentlichen Formulierungen werden durch Mischung des Prepolymers mit der Härterkomponente E) bzw. F) erhalten. In Komponente E) bzw. F) kann sich auch eine biologisch aktive Komponente D) befinden. Das Verhältnis von NCO-reaktiven NH-Gruppen zu freien NCO-Gruppen beträgt bevorzugt 1:1,5 bis 1:1, besonders bevorzugt 1:1.

Die erfindungswesentlichen Formulierungen umfassend oder bestehend aus den Komponenten D) sowie E) und/ oder F) besitzen unmittelbar nach Vermischung der Einzelkomponenten miteinander eine Scherviskosität bei 23°C von bevorzugt 1000 bis 10000 mPas, besonders bevorzugt 2000 bis 8000 mPas und ganz besonders bevorzugt 2500 bis 5000 mPas.

Die Geschwindigkeit bei 23°C bis eine vollständige Vernetzung und Aushärtung des Klebestoffs umfassend oder bestehend aus den Komponenten D) sowie E) und/ oder F) erreicht ist, beträgt typischerweise 30 s bis 10 min, bevorzugt 1 min bis 8 min.

Selbstverständlich können in die Gewebekleber auch pharmakologisch aktive Wirkstoffe wie Analgetika mit und ohne antiinflammatorische Wirkung, Antiphlogistika, antimikrobiell wirksame Substanzen, Antimykotika, antiparasitär wirkende Stoffe eingearbeitet sein.

Die Wirkstoffe können als reiner Wirkstoff oder aber in verkapselter Form vorliegen, um beispielsweise eine zeitlich verzögerte Abgabe zu erzielen. Als medizinische Wirkstoffe können im Rahmen der vorliegenden Erfindung eine Vielzahl von Wirkstofftypen und -klassen eingesetzt werden.

Ein solcher medizinischer Wirkstoff kann beispielsweise eine unter in vivo-Bedingungen Stickstoffmonoxid-freisetzende Komponente, bevorzugt L-Arginin oder eine L-Arginin-haltige oder eine L-Arginin freisetzende Komponente, besonders bevorzugt L-Arginin Hydrochlorid umfassen. Auch Prolin, Ornithin und/oder andere biogene Zwischenstufen wie beispielsweise biogene Polyamine (Spermin, Spermitin, Putrescin oder bioaktive künstliche Polyamine) können verwendet werden. Derartige Komponenten unterstützen bekanntermaßen die Wundheilung, wobei deren kontinuierliche mengenmäßig nahezu gleichmäßige Abgabe der Wundheilung besonders zuträglich ist.

Weitere erfindungsgemäß verwendbare Wirkstoffe umfassen mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Als Analgetika sind insbesondere nicht-steroidale Analgetika insbesondere Salicylsäure, Acetylsalicylsäure und deren Derivate z.B. Aspirin®, Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin®, Isopropy-Iphenazon und ganz besonders bevorzugt Arylessigsäuren sowie deren Derivate, Heteroarylessigsäuren sowie

deren Derivate, Arylpropionsäuren sowie deren Derivate und Herteroarylpropionsäuren sowie deren Derivate z.B. Indometacin®, Diclophenac®, Ibuprofen®, Naxoprophen®, Indomethacin®, Ketoprofen®, Piroxicam® geeignet.

Als Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor), TGF a; (Transforming Growth Factor alpha), TGF ß (Transforming Growth Factor beta), KGF (Keratinocyte Growth Factor), IGF1/IGF2 (Insulin-Like Growth Factor) und TNF (Tumor Necrosis Factor).

Als Vitamine oder Provitamine sind insbesondere die fettlöslichen oder wasserlöslichen Vitamine Vitamin A, Gruppe der Retinoide, Provitamin A, Gruppe der Carotenoide, insbesondere β-Carotin, Vitamin E, Gruppe der Tocopherole, insbesondere α Tocopherol, ß-Tocopherol, γ-Tocopherol, δ-Tocopherol und α-Tocotrienol, β-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B 1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist ein solches Mittel zu verwenden, das gemizid, bakterizid, bakteriostatisch, fungizid, viruzid, virustatisch und/ oder allgemein mikrobiozid wirkt.

Insbesondere sind solche Stoffe geeignet die ausgewählt werden aus der Gruppe Resorcinol, Iod, Iod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobiellen Metalle zu verwenden. Als antimikrobielle Metalle können insbesondere Silber, Kupfer oder Zink sowie deren Salze, Oxide oder Komplexe in Kombination oder alleine verwendet werden.

Als pflanzliche, wundheilungsfördernde Wirkstoffe sind im Zusammenhang mit der vorliegenden Erfindung insbesondere Extrakte der Kamille, Hamamelis-Extrakte z.B. Hamamelis virgina, Calendula-Extrakt, Aloe- Extrakt z.B. Aloe vera, Aloe barbadensis, Aloe feroxoder oder Aloe vulgaris, Grüntee- Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu nennen.

Der Gehalt der Wirkstoffe richtet sich dabei in erster Linie an der medizinisch erforderlichen Dosis aus sowie auch an der Verträglichkeit mit den übrigen Bestandteilen des erfindungsgemäßen Gewebeklebers.

Die Erfindung betrifft weiterhin ein Verfahren zur Erzeugung einer bakteriellen Barriere bei der Wundbehandlung, insbesondere nach chirurgischen Eingriffen, mittels eines erfindungsgemäßen Gewebeklebers, wobei das Verfahren die folgenden Schritte umfasst:

a) Zusammenführen der Wundränder der Wunde,;

b) Optionales Erzeugen einer Stütznaht aus chirurgischem Nahtmaterial, mit der die Wundränder miteinander verbunden werden;

c) Vermischen der ersten und zweiten Komponente des Zweikomponenten-Klebstoffs und Auftragen des Klebstoffs zumindest auf die Öffnungsstelle der den Bereich der zusammengefügten Enden der Wunde;

d) Aushärten lassen des Gewebeklebers.

e) Optionales anlegen eines Wundverbandes bei äußerer Anwendung.

Die Erfindung betrifft des Weiteren ein Kit enthaltend oder bestehend aus einem erfindungsgemäßen Gewebekleber und chirurgischem Nahtmaterial sowie optional einer Verfahrensanleitung zur Erzeugung einer bakterielle Barriere bei der Wundbehandlung, insbesondere nach chirurgischen Eingriffen, die das vorstehend genannte erfindungsgemäße Verfahren beschreibt. Hierfür kommen im Prinzip sämtliche dem Fachmann bekannten chirurgische Nahtmaterialien in Frage. Mit diesem Material können die zu verbindenden Gewebeteile zusätzlich zum Klebstoff stabilisiert werden. Dazu wird beispielsweise eine Stütznaht aus dem chirurgischem Nahtmaterial erzeugt. Diese Maßnahme wird zweckmäßigerweise vor dem Auftrag des Klebstoffs vorgenommen.

Bevorzugt liegen bei dem erfindungsgemäßen Kit die beiden Komponenten des Gewebeklebers in separaten Kammern einer Zweikammerspritze vor, wobei die Zweikammerspritze auslassseitig mit einem Statikmischer und optional mit einer Auftragsdüse versehen ist, wobei die Auftragsdüse insbesondere eine Breitschlitzdüse ist.

Die Erfindung betrifft insbesondere die folgenden Ausführungsformen:

Nach einer ersten Ausführungsform betrifft die Erfindung einen Gewebekleber in Form eines Zweikomponenten-Klebstoffs mit wenigstens einem aminofunktionellen Asparaginsäureester als Härter als erste Komponente und einem isocyanatfunktionellen Präpolymer als zweite Komponente zur Verwendung als bakterielle Barriere bei der Wundbehandlung, insbesondere nach chirurgischen Eingriffen.

Nach einer zweiten Ausführungsform betrifft die Erfindung einen Gewebekleber nach Ausführungsform 1, dadurch gekennzeichnet, dass der Gewebekleber folgende Komponenten umfasst oder daraus besteht:

ein isocyanatfunktionelles Präpolymer als Komponente A), erhältlich durch

a) Umsetzung einer wenigstens ein Zerewitinoff-aktives H-Atom aufweisenden H-funktionellen Starterverbindung mit einer Alkylenoxidverbindung und einem Co-Monomer zu einer Hydroxylgruppen tragenden Vorstufe, wobei das Co-Monomer ausgewählt ist aus der Gruppe umfassend Lactide, Glycolide, sowie Kombinationen hiervon und Kombinationen von Lactiden und / oder Glycoliden mit cyclischen Dicarbonsäureanhydriden und wobei das Co-Monomer durch eine statistische Copolymerisation in die Polymerkette(n) der Hydroxylgruppen tragenden Vorstufe eingebaut ist, sowie
b) Umsetzung der Hydroxylgruppen tragenden Vorstufe aus Schritt a) mit einem polyfunktionellen Isocyanat zu dem isocyanatfunktionellen Präpolymer,

ein aminofunktioneller Asparaginsäureester als Komponente B) der allgemeinen Formel (I)

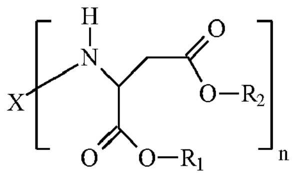
(I)

ist, wobei

X ein n-wertiger organischer Rest ist,
R1, R2 gleiche oder verschiedene organische Reste sind, die keine Zerewitinoff-aktiven H-Atome aufweisen,
n eine ganze Zahl ≥ 2 ist,

sowie optional ein Umsetzungsprodukt des isocyanatfunktionellen Präpolymers A) mit dem aminofunktionellen Asparaginsäureester B) als Komponente C).

Nach einer dritten Ausführungsform betrifft die Erfindung einen Gewebekleber nach Ausführungsform 2, dadurch gekennzeichnet, dass der aminofunktionelle Asparaginsäureester ein solcher der allgemeinen Formel (II)

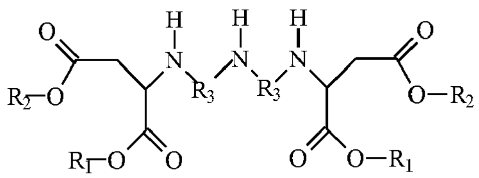
(II)

ist,
wobei R1, R2, R3 gleiche oder verschiedene organische Reste sind, die keine Zerewitinoff-aktiven H-Atome aufweisen.
Nach einer vierten Ausführungsform betrifft die Erfindung einen Gewebekleber nach Ausführungsform 2 oder 3, dadurch gekennzeichnet, dass das zur Herstellung der Hydroxylgruppen tragenden Vorstufe in Schritt a) eingesetzte Co-Monomer ein Laktid ist, bevorzugt Dilaktid, und der Gehalt an Laktidgruppen in der Hydroxylgruppen tragenden Vorstufe 2 bis 22 Gew.-% beträgt, vorzugsweise 3 bis 15 Gew.-%, weiter bevorzugt 4 bis 10 Gew.-%, besonders bevorzugt 6 bis 9 Gew.-%, wobei der Gehalt an Laktidgruppen in der Hydroxylgruppen tragenden Vorstufe wie in der Beschreibung ausgeführt definiert ist.
Nach einer fünften Ausführungsform betrifft die Erfindung einen Gewebekleber nach einer der Ausführungsformen 2 bis 4, dadurch gekennzeichnet, dass die Alkylenoxidverbindung aus Ethylenoxid und/ oder Propylenoxid ausgewählt ist, wobei der Anteil an Ethylenoxideinheiten in der Polymerkette der Hydroxylgruppen tragenden Vorstufe wenigstens 40 Gew.-% beträgt.
Nach einer sechsten Ausführungsform betrifft die Erfindung einen Gewebekleber nach einer der Ausführungsformen 2 bis 5, dadurch gekennzeichnet, dass das polyfunktionelle Isocyanat ausgewählt ist aus aliphatischen Isocyanaten.
Nach einer siebten Ausführungsform betrifft die Erfindung einen Gewebekleber nach einer der vorstehenden Ausführungsformen, dadurch gekennzeichnet, dass der Gewebekleber wenigstens einen weiteren Härter umfasst, der

insbesondere aus Polyolen mit einer zahlenmittleren Molmasse von 1000 Da oder weniger ausgewählt ist, insbesondere von 600 Da oder weniger.

Nach einer achten Ausführungsform betrifft die Erfindung einen Gewebekleber nach Ausführungsform 1, dadurch gekennzeichnet, dass der Gewebekleber die folgende Formulierung aus Komponenten D) sowie E) und/ oder F) umfasst oder daraus besteht:

D) isocyanatfunktionelle Prepolymere erhältlich aus

D1) aliphatischen Isocyanaten und

D2) Polyolen mit zahlenmittleren Molekulargewichten von ≥ 400 g/mol und mittleren OH-Funktionalitäten von 2 bis 6,

E) eine Härterkomponente umfassend

E1) aminofunktionelle Asparaginsäureester der allgemeinen Formel (III)

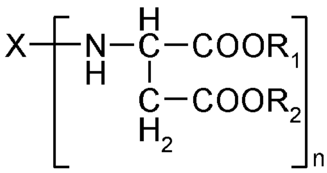

(III)

wobei

X ein n-wertiger organischer Rest ist, der durch Entfernung der primären Aminogruppen eines n-wertigen Amins erhalten wird,

$R_1$, $R_2$ gleiche oder verschiedene organische Reste sind, die keinen Zerewitinoff-aktiven Wasserstoff aufweisen und

n eine ganze Zahl von mindestens 2 ist

und gegebenenfalls

E2) organische Füllstoffe, die eine nach DIN 53019 gemessenen Viskosität bei 23°C im Bereich von 10 bis 6000 mPas aufweisen,

F) Umsetzungsprodukte von isocyanatfunktionellen Prepolymeren gemäß der Definition der Komponente D) mit Asparaginsäureestern gemäß Komponente E1) und/oder organischen Füllstoffen gemäß Komponente E2).

Nach einer neunten Ausführungsform betrifft die Erfindung einen Gewebekleber nach Ausführungsform 8, dadurch gekennzeichnet, dass in E2) Polyole eingesetzt werden, die zahlenmittlere Molekulargewichte von 4000 bis 8500 g/mol aufweisen.

Nach einer zehnten Ausführungsform betrifft die Erfindung einen Gewebekleber nach Ausführungsform 8 oder 9, dadurch gekennzeichnet, dass in E2) Polyalkylenoxid-Polyether, insbesondere Polyetherpolyole, eingesetzt werden.

Nach einer elften Ausführungsform betrifft die Erfindung einen Gewebekleber nach einer der Ausführungsformen 8 bis 10, dadurch gekennzeichnet, dass der Polyalkylenoxid-Polyether einen Gehalt an Ethylenoxid-basierten Einheiten von 60 bis 90 % bezogen auf die insgesamt enthaltene Mengen an Alkylenoxideinheiten aufweist.

Nach einer zwölften Ausführungsform betrifft die Erfindung einen Gewebekleber nach einer der Ausführungsformen 8 bis 11, dadurch gekennzeichnet, dass anstatt von der Härterkomponente E) ausschließlich zur Härtung der in D) eingesetzten Prepolymere die Umsetzungsprodukte gemäß F) eingesetzt werden.

Nach einer dreizehnten Ausführungsform betrifft die Erfindung einen Gewebekleber nach einer der vorstehenden Ausführungsformen, dadurch gekennzeichnet, dass der Gewebekleber zumindest einen pharmakologisch aktiven Wirkstoff enthält, ausgewählt aus der Gruppe umfassend Analgetika mit und ohne antiinflammatorische Wirkung, Antiphlogistika, antimikrobiell wirksame Substanzen, Antimykotika, antiparasitär wirkende Stoffe oder Mischungen von diesen, wobei die Wirkstoffe insbesondere als reiner Wirkstoff oder in verkapselter Form vorliegen.

Nach einer vierzehnten Ausführungsform betrifft die Erfindung einen Gewebekleber nach Ausführungsform 13, dadurch gekennzeichnet, dass der Wirkstoff ausgewählt ist aus

unter in vivo-Bedingungen Stickstoffmonoxid-freisetzende Komponenten, bevorzugt L-Arginin oder eine L-Arginin-haltige oder eine L-Arginin freisetzende Komponente, besonders bevorzugt L-Arginin Hydrochlorid,

Prolin, Ornithin und/oder biogenen Polyaminen, insbesondere Spermin, Spermitin, Putrescin oder bioaktiven künstlichen Polyaminen,

Vitaminen oder Provitaminen, Carotinoiden, Analgetika, Antiseptika, Hämostyptika, Antihistaminika, antimikrobiellen Metallen oder deren Salzen, pflanzlichen wundheilungsfördernden Substanzen oder Substanzgemischen, Pflanzenextrakten, Enzymen, Wachstumsfaktoren, Enzyminhibitoren sowie Kombinationen hiervon.

Nach einer fünfzehnten Ausführungsform betrifft die Erfindung ein Verfahren zur Erzeugung einer bakteriellen Barriere bei der Wundbehandlung, insbesondere nach chirurgischen Eingriffen, mittels eines Gewebeklebers nach einer der Ausführungsformen 1 bis 14, wobei das Verfahren die folgenden Schritte umfasst:

a) Zusammenführen der Wundränder der Wunde,;
b) Optionales Erzeugen einer Stütznaht aus chirurgischem Nahtmaterial, mit der die Wundränder miteinander verbunden werden;
c) Vermischen der ersten und zweiten Komponente des Zweikomponenten-Klebstoffs und Auftragen des Klebstoffs zumindest auf die Öffnungsstelle der den Bereich der zusammengefügten Enden der Wunde;
d) Aushärten lassen des Gewebeklebers.
e) Optionales anlegen eines Wundverbandes bei äußerer Anwendung.

Nach einer sechzehnten Ausführungsform betrifft die Erfindung ein Kit enthaltend oder bestehend aus einem Gewebekleber nach einer der Ausführungsformen 1 bis 14 und chirurgischem Nahtmaterial sowie optional einer Verfahrensanleitung zur Erzeugung einer bakterielle Barriere bei der Wundbehandlung, insbesondere nach chirurgischen Eingriffen, die das Verfahren gemäß Ausführungsform 15 beschreibt.

Nach einer siebzehnten Ausführungsform betrifft die Erfindung ein Kit nach Ausführungsform 16, dadurch gekennzeichnet, dass die beiden Komponenten des Gewebeklebers in separaten Kammern einer Zweikammerspritze vorliegt, wobei die Zweikammerspritze auslassseitig mit einem Statikmischer und optional mit einer Auftragsdüse versehen ist, wobei die Auftragsdüse insbesondere eine Breitschlitzdüse ist.

**Beispiel:**

Die vorliegende Erfindung wird im Folgenden anhand von Ausführungsbeispielen näher erläutert.

Eingesetzte Materialien:

Gewebekleber 1 (Erfindungsgemäß): Zwei-Komponenten Gewebekleber entsprechend dem Beispiel 3 der EP 2 307 063 B1.

Gewebekleber 2, 3, 4 (Erfindungsgemäß): Zwei-Komponenten Gewebekleber basierend auf dem trifuktionellen Polyol 1 der EP 2 794 710 B1, wobei der Laktidgehalt in der Hydroxylgruppen tragenden Vorstufe der jeweiligen Gewebekleber durch Variation der Einsatzmenge von Dilactid auf die in der folgenden Tabelle angegeben Werte eingestellt wurde. Der Gehalt an Laktidgruppen in der Hydroxylgruppen tragenden Vorstufe ist wie folgt definiert:

Gehalt Laktid in der Hydroxylgruppen tragenden Vorstufe (in Gew.-%):

$$m(Dilaktid)*100/[m(Dilaktid)+m(Kat)+m(EO)+m(PO)+m(Starter)]$$

Kat = Katalysator, EO = Ethylenoxid, PO = Propylenoxid; Starter = Starterverbindung

Anschließend erfolgte die Umsetzung des jeweiligen Polyols zum Präpolymer 2, welches dann jeweils mit den Aspartaten A und B sowie PEG 200 im folgenden Mischungsverhältnis (Aspartat A/Aspartat B/PEG 200; 0,369/0,098/0,53) formuliert wurde.

Die erfindungsgemäßen Gewebekleber 1 - 4 wurden jeweils mit einer Zweikammerspritze mit aufgestecktem Statikmischer verwendet.

Gewebekleber 5 (Vergleich): Dermabond ® der Firma Ethicon US LLC, Somerville, New Jersey

Die Laktidgehalte in der Hydroxylgruppen tragenden Vorstufe der erfindungsgemäßen Gewebekleber waren wie folgt:

| | Laktidgehalt |
|---|---|
| Gewebekleber 1 | 0 Gew.-% |
| Gewebekleber 2 | 2 Gew.-% |
| Gewebekleber 3 | 5 Gew.-% |
| Gewebekleber 4 | 8 Gew.-% |

Versuchsdurchführung:

Die Barrierefunktion der Gewebekleber 1 bis 4 gegenüber Bakterien wurde mit Hilfe eines in vitro Assays nachgewiesen. Er beruht auf der Verwendung des Bakterium Escherichia coli lux (E. coli lux), dessen Position und Menge (angegeben in kolonieformenden Einheiten = colony forming units = CFU) über die Visualisierung und Messung ihrer natürliche Lumineszenz bestimmt werden kann. Die Lumineszenz der Bakterien ist dabei linear zur Menge der Bakterien und kann somit mit Hilfe einer Lumineszenz-Messung bestimmt werden.

Der jeweilige Gewebekleber wird unter Verwendung der Breitschlitzdüse auf einem sterilen Untergrund ausgedrückt. Nachdem er zu einem dünnen Film (ca. 2 mm dick) ausgehärtet ist, wurden 2 x 2 cm große Stücke zugeschnitten und auf Agar Platten platziert. Auf die oben liegende Seite des Gewebeklebers wurde 5 $\mu$L einer 1:1000 Verdünnung einer E. coli lux-Stammlösung mit einer Konzentration von ca. 1x105 oder 2x107 CFU/mL gegeben.

Die Agar Platten wurden bei 37 °C für 1, 24, 48 und 72 Stunde(n) inkubiert. Beim jeweiligen Zeitpunkt wurden das Bakterienwachstum und die Anhaftung des Kleberfilms und des Filterpapiers makroskopisch bewertet. Außerdem wurde die Menge der E. coli lux Bakterien mittels eines Lumineszenz Imaging Systems (IVIS System) bestimmt. Dabei wurden drei verschiedene Ebenen beurteilt: Aufsicht, Stelle der Anhaftung nach Entfernen des Kleberfilms oder Filterpapiers und ein Querschnitt der Proben.

| Gewebekleber: Stunden | 1 (Erf.) | 2 (Erf.) | 3 (Erf.) | 4 (Erf.) | 5 (Vgl.) |
|---|---|---|---|---|---|
| 24 h | | | 98,72% | 99,7% | 99,99% |
| 48 h | 100% | 44,28% | 86,11% | 98,87% | 99,98% |
| 72 h | >99% | 16,09% | 62,88% | 96,52% | 99,99% |

Die Prozentangaben der Barrierefunktion gegenüber Bakterien stehen im Vergleich zur Kontrolle mit Filterpapier.

Die Resultate des Gewebeklebers 1 ergaben eine Barrierefunktion bis zu 48 Stunden ohne Penetration der Bakterien durch den Gewebekleber. Nach 72 Stunden wurde eine geringe Bakterienkonzentration auf der Agarplatte nachgewiesen.

Die Resultate insgesamt zeigen, dass alle verwendeten Gewebekleber eine bakterielle Barrierefunktion besitzen. Die Barrierefunktion der Gewebekleber 1, 3 und 4 sind zudem sogar vergleichbar zur Barrierefunktion des Vergleichsklebers 5 (Dermabond®).

## Patentansprüche

1. Gewebekleber in Form eines Zweikomponenten-Klebstoffs mit wenigstens einem aminofunktionellen Asparaginsäureester als Härter als erste Komponente und einem isocyanatfunktionellen Präpolymer als zweite Komponente zur Verwendung als bakterielle Barriere bei der Wundbehandlung, insbesondere nach chirurgischen Eingriffen.

2. Gewebekleber nach Anspruch 1, **dadurch gekennzeichnet, dass** der Gewebekleber folgende Komponenten umfasst oder daraus besteht:

   ein isocyanatfunktionelles Präpolymer als Komponente A), erhältlich durch

   a) Umsetzung einer wenigstens ein Zerewitinoff-aktives H-Atom aufweisenden H-funktionellen Starterverbindung mit einer Alkylenoxidverbindung und einem Co-Monomer zu einer Hydroxylgruppen tragenden Vorstufe, wobei das Co-Monomer ausgewählt ist aus der Gruppe umfassend Lactide, Glycolide, sowie

Kombinationen hiervon und Kombinationen von Lactiden und / oder Glycoliden mit cyclischen Dicarbonsäureanhydriden und wobei das Co-Monomer durch eine statistische Copolymerisation in die Polymerkette(n) der Hydroxylgruppen tragenden Vorstufe eingebaut ist, sowie

b) Umsetzung der Hydroxylgruppen tragenden Vorstufe aus Schritt a) mit einem polyfunktionellen Isocyanat zu dem isocyanatfunktionellen Präpolymer,

ein aminofunktioneller Asparaginsäureester als Komponente B) der allgemeinen Formel (I)

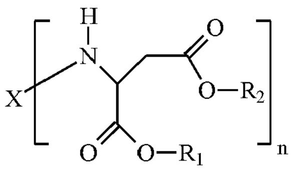
(I)

ist, wobei

X ein n-wertiger organischer Rest ist,
$R_1$, $R_2$ gleiche oder verschiedene organische Reste sind, die keine Zerewitinoff-aktiven H-Atome aufweisen,
n eine ganze Zahl ≥ 2 ist,

sowie optional ein Umsetzungsprodukt des isocyanatfunktionellen Präpolymers A) mit dem aminofunktionellen Asparaginsäureester B) als Komponente C).

3. Gewebekleber nach Anspruch 2, **dadurch gekennzeichnet, dass** der aminofunktionelle Asparaginsäureester ein solcher der allgemeinen Formel (II)

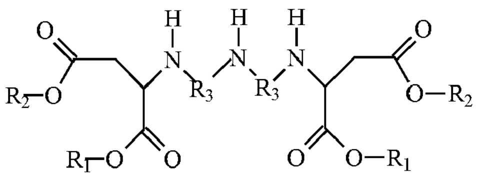
(II)

ist, wobei R1, R2, R3 gleiche oder verschiedene organische Reste sind, die keine Zerewitinoff-aktiven H-Atome aufweisen.

4. Gewebekleber nach Anspruch 2 oder 3, **dadurch gekennzeichnet, dass** das zur Herstellung der Hydroxylgruppen tragenden Vorstufe in Schritt a) eingesetzte Co-Monomer ein Laktid ist, bevorzugt Dilaktid, und der Gehalt an Laktidgruppen in der Hydroxylgruppen tragenden Vorstufe 2 bis 22 Gew.-% beträgt, vorzugsweise 3 bis 15 Gew.-%, weiter bevorzugt 4 bis 10 Gew.-%, besonders bevorzugt 6 bis 9 Gew.-%, wobei der Gehalt an Laktidgruppen in der Hydroxylgruppen tragenden Vorstufe wie in der Beschreibung ausgeführt definiert ist.

5. Gewebekleber nach einem der Ansprüche 2 bis 4, **dadurch gekennzeichnet, dass** die Alkylenoxidverbindung aus Ethylenoxid und/ oder Propylenoxid ausgewählt ist, wobei der Anteil an Ethylenoxideinheiten in der Polymerkette der Hydroxylgruppen tragenden Vorstufe wenigstens 40 Gew.-% beträgt.

6. Gewebekleber nach einem der Ansprüche 2 bis 5, **dadurch gekennzeichnet, dass** das polyfunktionelle Isocyanat ausgewählt ist aus aliphatischen Isocyanaten.

7. Gewebekleber nach einem der vorstehenden Ansprüche, **dadurch gekennzeichnet, dass** der Gewebekleber wenigstens einen weiteren Härter umfasst, der insbesondere aus Polyolen mit einer zahlenmittleren Molmasse von 1000 Da oder weniger ausgewählt ist, insbesondere von 600 Da oder weniger.

8. Gewebekleber nach Anspruch 1, **dadurch gekennzeichnet, dass** der Gewebekleber die folgende Formulierung aus Komponenten D) sowie E) und/ oder F) umfasst oder daraus besteht:

D) isocyanatfunktionelle Prepolymere erhältlich aus

D1) aliphatischen Isocyanaten und
D2) Polyolen mit zahlenmittleren Molekulargewichten von ≥ 400 g/mol und mittleren OH-Funktionalitäten von 2 bis 6,

E) eine Härterkomponente umfassend

E1) aminofunktionelle Asparaginsäureester der allgemeinen Formel (III)

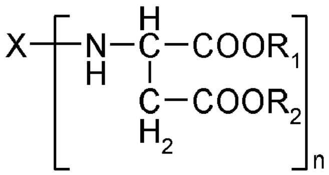

(III)

wobei

X ein n-wertiger organischer Rest ist, der durch Entfernung der primären Aminogruppen eines n-wertigen Amins erhalten wird,
$R_1$, $R_2$ gleiche oder verschiedene organische Reste sind, die keinen Zerewitinoff-aktiven Wasserstoff aufweisen und
n eine ganze Zahl von mindestens 2 ist

und gegebenenfalls
E2) organische Füllstoffe, die eine nach DIN 53019 gemessenen Viskosität bei 23°C im Bereich von 10 bis 6000 mPas aufweisen,

F) Umsetzungsprodukte von isocyanatfunktionellen Prepolymeren gemäß der Definition der Komponente D) mit Asparaginsäureestern gemäß Komponente E1) und/oder organischen Füllstoffen gemäß Komponente E2).

**9.** Gewebekleber nach Anspruch 8, **dadurch gekennzeichnet, dass** in E2) Polyole eingesetzt werden, die zahlenmittlere Molekulargewichte von 4000 bis 8500 g/mol aufweisen.

**10.** Gewebekleber nach Anspruch 8 oder 9, **dadurch gekennzeichnet, dass** in E2) Polyalkylenoxid-Polyether, insbesondere Polyetherpolyole, eingesetzt werden.

**11.** Gewebekleber nach einem der Ansprüche 8 bis 10, **dadurch gekennzeichnet, dass** der Polyalkylenoxid-Polyether einen Gehalt an Ethylenoxid-basierten Einheiten von 60 bis 90 % bezogen auf die insgesamt enthaltene Mengen an Alkylenoxideinheiten aufweist.

**12.** Gewebekleber nach einem der Ansprüche 8 bis 11, **dadurch gekennzeichnet, dass** anstatt von der Härterkomponente E) ausschließlich zur Härtung der in D) eingesetzten Prepolymere die Umsetzungsprodukte gemäß F) eingesetzt werden.

**13.** Verfahren zur Erzeugung einer bakteriellen Barriere bei der Wundbehandlung, insbesondere nach chirurgischen Eingriffen, mittels eines Gewebeklebers nach einem der Ansprüche 1 bis 12, wobei das Verfahren die folgenden Schritte umfasst:

a) Zusammenführen der Wundränder der Wunde,;
b) Optionales Erzeugen einer Stütznaht aus chirurgischem Nahtmaterial, mit der die Wundränder miteinander verbunden werden;
c) Vermischen der ersten und zweiten Komponente des Zweikomponenten-Klebstoffs und Auftragen des Klebstoffs zumindest auf die Öffnungsstelle der den Bereich der zusammengefügten Enden der Wunde;
d) Aushärten lassen des Gewebeklebers.
e) Optionales anlegen eines Wundverbandes bei äußerer Anwendung.

**14.** Kit enthaltend oder bestehend aus einem Gewebekleber nach einem der Ansprüche 1 bis 12 und chirurgischem Nahtmaterial sowie optional einer Verfahrensanleitung zur Erzeugung einer bakterielle Barriere bei der Wundbehandlung, insbesondere nach chirurgischen Eingriffen, die das Verfahren gemäß Anspruch 13 beschreibt.

**15.** Kit nach Anspruch 14, **dadurch gekennzeichnet, dass** die beiden Komponenten des Gewebeklebers in separaten Kammern einer Zweikammerspritze vorliegt, wobei die Zweikammerspritze auslassseitig mit einem Statikmischer und optional mit einer Auftragsdüse versehen ist, wobei die Auftragsdüse insbesondere eine Breitschlitzdüse ist.

Europäisches Patentamt
European Patent Office
Office européen des brevets

# EUROPÄISCHER RECHERCHENBERICHT

Nummer der Anmeldung
EP 21 17 9463

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | EP 2 098 254 A1 (BAYER MATERIALSCIENCE AG [DE]) 9. September 2009 (2009-09-09)<br>* Absätze [0013] - [0027], [0064] - [0069], [0077], [0085], [0086]; Ansprüche 1-13; Beispiele 1-4 *<br>----- | 1-13<br>14,15 | INV.<br>A61L24/00<br>A61L24/04 |
| Y | WO 2021/074080 A1 (ADHESYS MEDICAL GMBH [DE]) 22. April 2021 (2021-04-22)<br>* Seiten 2,5,6; Ansprüche 1-15; Beispiel 1 *<br>----- | 14,15 | |
| A,D | EP 2 794 710 B1 (ADHESYS MEDICAL GMBH [DE]) 15. August 2018 (2018-08-15)<br>* Ansprüche 1-10 *<br>----- | 1-15 | |
| A | WO 2013/092506 A1 (BAYER MATERIALSCIENCE AG [DE]) 27. Juni 2013 (2013-06-27)<br>* Ansprüche 1-20 *<br>----- | 1-15 | |
| | | | RECHERCHIERTE SACHGEBIETE (IPC)<br>A61L |

1 Der vorliegende Recherchenbericht wurde für alle Patentansprüche erstellt

| Recherchenort | Abschlußdatum der Recherche | Prüfer |
|---|---|---|
| Den Haag | 2. Dezember 2021 | Burtan, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 21 17 9463

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-12-2021

| Im Recherchenbericht angeführtes Patentdokument | Datum der Veröffentlichung | Mitglied(er) der Patentfamilie | Datum der Veröffentlichung |
|---|---|---|---|
| EP 2098254 A1 | 09-09-2009 | AU 2009221268 A1 | 11-09-2009 |
| | | BR PI0909101 A2 | 11-08-2015 |
| | | CA 2717761 A1 | 11-09-2009 |
| | | CN 101959541 A | 26-01-2011 |
| | | EP 2098254 A1 | 09-09-2009 |
| | | EP 2262546 A1 | 22-12-2010 |
| | | ES 2396279 T3 | 20-02-2013 |
| | | JP 2011514819 A | 12-05-2011 |
| | | KR 20100131444 A | 15-12-2010 |
| | | RU 2010140659 A | 20-04-2012 |
| | | TW 200950826 A | 16-12-2009 |
| | | US 2011003898 A1 | 06-01-2011 |
| | | WO 2009109306 A1 | 11-09-2009 |
| WO 2021074080 A1 | 22-04-2021 | EP 3808385 A1 | 21-04-2021 |
| | | WO 2021074080 A1 | 22-04-2021 |
| EP 2794710 B1 | 15-08-2018 | CN 104105731 A | 15-10-2014 |
| | | CN 108570140 A | 25-09-2018 |
| | | CY 1121022 T1 | 11-12-2019 |
| | | DK 2794710 T3 | 10-12-2018 |
| | | DK 3385294 T3 | 08-02-2021 |
| | | EP 2794710 A1 | 29-10-2014 |
| | | EP 3385294 A1 | 10-10-2018 |
| | | EP 3786207 A1 | 03-03-2021 |
| | | ES 2694826 T3 | 27-12-2018 |
| | | ES 2846300 T3 | 28-07-2021 |
| | | HK 1201547 A1 | 04-09-2015 |
| | | HR P20181653 T1 | 14-12-2018 |
| | | HU E040031 T2 | 28-02-2019 |
| | | JP 6097764 B2 | 15-03-2017 |
| | | JP 2015508426 A | 19-03-2015 |
| | | LT 2794710 T | 26-11-2018 |
| | | PL 2794710 T3 | 28-02-2019 |
| | | PT 2794710 T | 22-11-2018 |
| | | SI 2794710 T1 | 31-12-2018 |
| | | US 2014316076 A1 | 23-10-2014 |
| | | US 2016256595 A1 | 08-09-2016 |
| | | WO 2013092504 A1 | 27-06-2013 |
| WO 2013092506 A1 | 27-06-2013 | CN 104144999 A | 12-11-2014 |
| | | DK 2794798 T3 | 29-02-2016 |
| | | EP 2794798 A1 | 29-10-2014 |
| | | ES 2560031 T3 | 17-02-2016 |
| | | HK 1201550 A1 | 04-09-2015 |
| | | JP 5873931 B2 | 01-03-2016 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR. EP 21 17 9463

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-12-2021

| Im Recherchenbericht angeführtes Patentdokument | Datum der Veröffentlichung | Mitglied(er) der Patentfamilie | Datum der Veröffentlichung |
|---|---|---|---|
| | | JP 2015509117 A | 26-03-2015 |
| | | US 2014341835 A1 | 20-11-2014 |
| | | US 2017119921 A1 | 04-05-2017 |
| | | WO 2013092506 A1 | 27-06-2013 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2794710 B1
- EP 2307063 B1
- EP 2145634 B1
- EP 1525244 A1
- EP 2046861 A
- DE 69311633 A